# EUROPEAN PATENT APPLICATION

(11) **EP 4 538 362 A1**
(43) Date of publication of application: **16.04.2025**
(21) Application number: 23819823.8
(22) Date of filing: 06.06.2023
(51) Int. Cl.: C12N 1/21, C12N 15/54, C12P 19/18, C12N 9/10

(54) **MICROORGANISM HAVING IMPROVED 3'-SIALYLLACTOSE PRODUCTIVITY AND PRODUCTION METHOD OF 3'-SIALYLLACTOSE**

(30) Priority: 06.06.2022 JP 2022091801
(71) Applicant: Kyowa Hakko Bio Co., Ltd., Tokyo 100-8185 (JP)
(72) Inventor: NAOE, Taiki, Tokyo 100-8185 (JP); UJIHARA, Tetsuro, Tokyo 100-8185 (JP); AOKI, Yuriko, Tokyo 100-8185 (JP)
(74) Representative: Grünecker Patent- und Rechtsanwälte PartG mbB
(86) International application number: PCT/JP2023/020924
(87) International publication number: WO 2023/238843

(57) **Abstract**

The present invention relates to a microorganism which has an enhanced activity of a protein consisting of an amino acid sequence derived from the amino acid sequence represented by SEQ ID NO: 2 by deletion of 20-37 amino acid residues on the N-terminal side, and which has an improved productivity of 3'-sialyllactose compared to the parent strain.

## Description

### TECHNICAL FIELD

The present invention relates to a microorganism having improved productivity of 3'-sialyllactose and a method for producing 3'-sialyllactose.

### BACKGROUND ART

3'-Sialyllactose (hereinafter, referred to as 3'SL) is acidic oligosaccharide contained in human milk, and together with 6'-sialyllactose, is a main acidic human milk oligosaccharide (Patent Literature 1). 3'SL is known to play an important role in language development, cognitive function, and various health conditions of infants (Non Patent Literatures 2, 3, and 4).

3'SL is contained in human milk, but is contained in small amounts in milk of other mammals, such as cow milk (Non Patent Literature 5). Infant milk is mainly produced by processing a raw material such as cow milk with necessary nutrients, so that a content of human milk oligosaccharides, including 3'SL, is lower than that in human milk. Therefore, it is desirable to add 3'SL to infant milk in order to give infant milk the same effects as human milk (Non Patent Literature 6).

Non Patent Literature 6 discloses various methods for acquiring a human milk oligosaccharide, such as an extraction method, a chemical synthesis method, and a fermentation method using a recombinant microorganism. The fermentation method using a recombinant microorganism is considered to be the most economically rational method among them.

As disclosed in Non Patent Literature 7 and Patent Literature 1, a typical method for producing 3'SL using a recombinant microorganism involves synthesizing cytidine-5'-monophosphate (hereinafter, also referred to as "CMP")-sialic acid from an inexpensive carbon source such as glycerol or glucose within cells, and transferring a sialic acid residue of the CMP-sialic acid to lactose added from the outside using an α2,3-sialyltransferase to produce 3'SL.

### CITATION LIST

### PATENT LITERATURE

Patent Literature 1: WO2015/037698

### NON-PATENT LITERATURE

Non Patent Literature 1: Nutrition Reviews 75 (2017) 920-933
Non Patent Literature 2: The American Journal of Clinical Nutrition 114 (2021) 588-597
Non Patent Literature 3: Nutrients 13 (2021) 4191
Non Patent Literature 4: Nature Metabolism 2 (2020) 678-687
Non Patent Literature 5: Journal of Dairy Science 86 (2003) 52-59
Non Patent Literature 6: Nutrition Review 74 (2016) 635-644
Non Patent Literature 7: Journal of Biotechnology 134 (2008) 261-265
Non Patent Literature 8: Journal of Biological Chemistry 271 (1996) 28271-28276
Non Patent Literature 9: Journal of Biological Chemistry 275 (2000) 3896-3906
Non Patent Literature 10: Journal of Applied Glycoscience 56 (2009) 77-82
Non Patent Literature 11: Journal of Biological Chemistry 282 (2007) 29274-29802
Non Patent Literature 12: Journal of the American Chemical Society 127 (2005) 17618-17619
Non Patent Literature 13: Molecular Microbiology 39 (2001) 341-350
Non Patent Literature 14: Biotechnology Letters 30 (2008) 671-676
Non Patent Literature 15: Journal of Biological Chemistry 286 (2011) 37237-37248

### SUMMARY OF INVENTION

### TECHNICAL PROBLEM

In the production method using a recombinant microorganism, an activity of an α2,3-sialyltransferase, which transfers a sialic acid residue to lactose, is thought to be important for productivity of 3'SL. Various types of α2,3-sialyltransferases have already been cloned from microorganisms, and α2,3-sialyltransferases have been successfully expressed in Escherichia coli and the like (Non Patent Literatures 8, 9, 10, 11, 12, 13). In order to improve the productivity of 3'SL as compared with a method in related art, there is a demand for an α2,3-sialyltransferase that can transfer a sialic acid residue to lactose with high activity and efficiency.

Sialyltransferases are generally known to have a region at the N-terminus of the amino acid sequence that is not associated with an activity. Non Patent Literatures 14 and 15 disclose a method for expressing an exogenous sialyltransferase in Escherichia coli by cleaving the N-terminus of the amino acid sequence. According to the method, it has been known that the expression and activity of the sialyltransferase change, but it has not been clarified which region is specifically cleaved to obtain a useful activity.

Therefore, an object of the present invention is to provide a microorganism having an enhanced activity of a protein with a specific amino acid residue deleted and improved productivity of 3'SL as compared with that of a parent strain. An object of the present invention is to provide a method for producing 3'SL using the microorganism.

### SOLUTION TO PROBLEM

As a result of intensive studies, the present inventors have found that a microorganism having an enhanced activity of a protein consisting of an amino acid sequence in which 20 to 37 amino acid residues in the amino acid sequence represented by SEQ ID NO: 2 on an N-terminus side is deleted has improved productivity of 3'SL as compared with that of a parent strain, and thus completed the present invention.

That is, the present invention is as follows.
<1> A microorganism having an enhanced activity of a protein consisting of an amino acid sequence in which N-terminus 20 to 37 amino acid residues in the amino acid sequence represented by SEQ ID NO: 2 are deleted, where the microorganism has improved productivity of 3'-sialyllactose as compared with that of a parent strain.
<2> The microorganism according to the above <1>, in which the protein consisting of an amino acid sequence in which N-terminus 20 to 37 amino acid residues in the amino acid sequence represented by SEQ ID NO: 2 are deleted is a protein according to any one of the following [1] to [3]:
   [1] a protein consisting of the amino acid sequence represented by SEQ ID NO: 4, 6, 8, or 10,
   [2] a mutant protein having an α2,3-sialyltransferase activity and consisting of an amino acid sequence in which 1 to 20 amino acids are deleted, substituted, inserted, or added in the amino acid sequence represented by SEQ ID NO: 4, 6, 8, or 10, and
   [3] a homologous protein having an α2,3-sialyltransferase activity and consisting of an amino acid sequence having an identity of 90% or more with the amino acid sequence represented by SEQ ID NO: 4, 6, 8, or 10.
<3> A method for producing 3'-sialyllactose, including: preparing the microorganism according to the above <1> or <2>; and producing 3'-sialyllactose in a culture using the microorganism.

### ADVANTAGEOUS EFFECTS OF INVENTION

The microorganism according to one aspect of the present invention has improved 3'SL productivity by enhancing an activity of a protein with a specific amino acid residue deleted. According to the production method of one aspect of the present invention, 3'SL can be produced by using a culture obtained by culturing the microorganism.

### BRIEF DESCRIPTION OF DRAWINGS

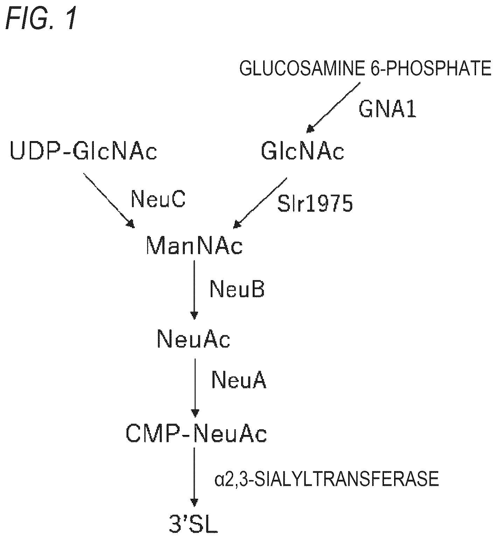

[FIG. 1] FIG. 1 is a diagram showing an example of a 3'SL biosynthetic pathway.

### DESCRIPTION OF EMBODIMENTS

### 1. Microorganism having Improved Productivity of 3'SL

Examples of a microorganism of one aspect of the present invention include a microorganism having an enhanced activity of a protein according to the following [A] and improved productivity of 3'SL as compared with that of a parent strain.
[A] A protein consisting of an amino acid sequence in which N-terminus 20 to 37 amino acid residues in the amino acid sequence represented by SEQ ID NO: 2 are deleted.

### <Productivity>

In the present description, the productivity refers to an ability to accumulate a target oligosaccharide produced by a microorganism in a culture of the microorganism. The productivity of an oligosaccharide by a microorganism can be confirmed by detecting an oligosaccharide in a culture of the microorganism using an analyzer to be described later.

It is preferable that the protein consisting of an amino acid sequence in which N-terminus 20 to 37 amino acid residues in the amino acid sequence represented by SEQ ID NO: 2 are deleted has an α2,3-sialyltransferase activity higher than that of a protein consisting of the amino acid sequence represented by SEQ ID NO: 2. The protein consisting of an amino acid sequence in which N-terminus 20 to 37 amino acid residues in the amino acid sequence represented by SEQ ID NO: 2 are deleted means a protein consisting only of an amino acid sequence in which N-terminus 20 to 37 amino acid residues in the amino acid sequence represented by SEQ ID NO: 2 are deleted.

The α2,3-sialyltransferase activity refers to an activity of using CMP-sialic acid and a receptor carbohydrate as a substrate, transferring a sialic acid residue to a terminus galactose of the carbohydrate via an α2,3 bond to obtain a sialic acid-containing carbohydrate.

Examples of the CMP-sialic acid is CMP-N-acetylneuraminic acid (hereinafter, also referred to as "CMP-NeuAc").

The receptor carbohydrate used as a substrate may be any of an oligosaccharide, a polysaccharide, and a complex carbohydrate such as a glycoprotein and a glycolipid as long as the receptor carbohydrate serves as a substrate for sialyllactose.

Examples of the oligosaccharide or polysaccharide serving as a substrate for sialyllactose include an oligosaccharide or polysaccharide having galactose at the non-reducing end, or an oligosaccharide or polysaccharide having N-acetylneuraminic acid (hereinafter, also referred to as "NeuAc") at the non-reducing end. Preferred examples thereof include an oligosaccharide having a structure at the non-reducing end selected from the group consisting of lactose, globotriose, N-acetyllactosamine, lacto-N-tetraose, lacto-N-neotetraose, Lewis a, and Lewis X, and more preferred examples thereof include lactose.

Examples of the complex carbohydrate include a complex carbohydrate in which a protein or a lipid is bound to the above-described oligosaccharide and polysaccharide.

As the sialic acid-containing carbohydrate, a NeuAc-containing carbohydrate is preferred. Examples of the NeuAc-containing carbohydrate include a carbohydrate in which NeuAc is added to the above-described receptor carbohydrate, preferably include a carbohydrate containing an oligosaccharide having NeuAcα2-3Galβ1-4Glc at the non-reducing end, and more preferably include 3'SL.

FIG. 1 shows an example of a 3'SL biosynthetic pathway. It is presumed that the enhanced α2,3-sialyltransferase activity of the microorganism promotes an α2,3 bond of a NeuAc residue to a galactose residue of lactose using CMP-NeuAc and lactose as a substrate, thereby improving the 3'SL productivity.

The protein consisting of an amino acid sequence in which N-terminus 20 to 37 amino acid residues in the amino acid sequence represented by SEQ ID NO: 2 are deleted according to the above [A] is a protein according to any one of the following [1] to [3].
[1] A protein consisting of the amino acid sequence represented by SEQ ID NO: 4, 6, 8, or 10.
[2] A mutant protein having an α2,3-sialyltransferase activity and consisting of an amino acid sequence in which 1 to 20 amino acids are deleted, substituted, inserted, or added in the amino acid sequence represented by SEQ ID NO: 4, 6, 8, or 10.
[3] A homologous protein having an α2,3-sialyltransferase activity and consisting of an amino acid sequence having an identity of 90% or more with the amino acid sequence represented by SEQ ID NO: 4, 6, 8, or 10.

The mutant protein refers to a protein obtained by artificially deleting or substituting an amino acid residue of an original protein or artificially inserting or adding an amino acid residue in the protein.

The expression "an amino acid is deleted, substituted, inserted, or added in the mutant protein of the above [2]" may mean that 1 to 20 amino acids are deleted, substituted, inserted, or added at any position in the same sequence. The number of amino acids to be deleted, substituted, inserted, or added is 1 to 20, preferably 1 to 10, further preferably 1 to 8, and most preferably 1 to 5.

The amino acid to be deleted, substituted, inserted, or added may be of a natural type or a non-natural type. Examples of the natural type amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

In the present description, the homologous protein is a protein whose encoding gene is thought to have the same evolutionary origin as a gene encoding an original protein due to similarity in structure and function with the original protein, and is a protein possessed by organisms in nature.

Examples of the homologous protein include an amino acid sequence having an identity of preferably 90% or more, and particularly preferably 95% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST program and Gapped BLAST programs are used, default parameters for each program are used. A specific method of the analysis methods is well known.

### <Parent Strain>

In the present invention, the parent strain refers to an original strain to be subjected to genetic modification, transformation, and the like. The original strain to be subjected to transformation by genetic transfer is also referred to as a host strain.

In the present description, examples of the parent strain preferably include a prokaryote or a yeast strain, more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and most preferably include a prokaryote such as Escherichia coli BW25113 (available from the National Institute of Genetics), Escherichia coli MG1655, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 codon plus (manufactured by Stratagene Corporation), Escherichia coli W3110S3GK (NBRC114657), Escherichia coli ATCC 9637, Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC 14066, Cory nebacterium ammoniagenes, Corynebacterium glutamicum ATCC 13032, Corynebacterium glutamicum ATCC 14067, Corynebacterium glutamicum ATCC 13869, Corynebacterium acetoacidophilum ATCC 13870, Microbacterium ammoniaphilum ATCC 15354, or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The parent strain may be a wild-type strain. When a wild-type strain does not have an ability to produce a substrate for 3'SL production, such as CMP-NeuAc and/or lactose, the parent strain may be a bred strain that is artificially imparted with the ability to produce CMP-NeuAc and/or lactose.

The production of CMP-NeuAc and/or lactose by a microorganism can be confirmed, for example, by culturing the microorganism in a medium and detecting CMP-NeuAc and/or lactose accumulated in the culture by a known method such as HPLC to be described later.

Examples of a method for artificially imparting or enhancing the ability to produce CMP-NeuAc and/or lactose include well-known methods such as the following (a) to (d). These methods can be used alone or in combination.
(a) A method for enhancing expression of at least one enzyme associated with a biosynthesis pathway for producing CMP-NeuAc and/or lactose
(b) A method for increasing the number of copies of at least one enzyme gene associated with a biosynthesis pathway for producing CMP-NeuAc and/or lactose
(c) A method for alleviating or releasing at least one mechanism for regulating a biosynthesis pathway for producing CMP-NeuAc and/or lactose
(d) A method for weakening or blocking at least one metabolic pathway branching off from a biosynthesis pathway for producing CMP-NeuAc and/or lactose to a metabolic product other than the target substance

### <<Preferred Activity to be Enhanced in Parent Strain>>

The parent strain preferably has at least one activity selected from an uridine diphosphate N-acetylglucosamine (hereinafter, also referred to as "UDP-GlcNAc") epimerase activity, a NeuAc synthase activity, a pyruvate carboxylase activity, an L-glutamine-D-fructose 6-phosphate aminotransferase activity, a NanT activity, a CMP-NeuAc synthase activity, a lactose permease activity, a glucosamine acetyltransferase activity, and an N-acetylmannosamine epimerase activity, and more preferably has an enhanced activity thereof. Among them, the parent strain preferably has a CMP-NeuAc synthase activity, and more preferably has an enhanced activity thereof.

UDP-GlcNAc epimerase is an enzyme responsible for a reaction of producing N-acetylmannosamine (hereinafter, also referred to as "ManNAc") from UDP-GlcNAc, and is encoded by the neuC gene. The neuC gene is preferaby derived from the Flavobacterium psychrophilum strain NBRC100250.

NeuAc synthase is an enzyme responsible for a reaction of producing NeuAc using ManNAc and phosphoenolpyruvate (hereinafter, also referred to as "PEP") as a substrate. Examples of a DNA encoding the NeuAc synthase is a DNA encoding CjneuB and preferably derived from a prokaryote such as a bacterium or yeast, particularly preferably derived from a prokaryote, and particularly preferably derived from Campylobacter jejuni ATCC 43438 strain or a DNA encoding neuB and derived from Rhodobacter capsulatus.

Pyruvate carboxylase is an enzyme responsible for a reaction of carboxylating pyruvate to produce oxaloacetic acid, and is encoded by the pyc gene. Since PEP is generated by decarboxylation of oxaloacetate, Pyc enhances supply of PEP. The pyc gene is preferably derived from Sinorhizobium meliloti or Corynebacterium glutamicum.

L-glutamine-D-fructose-6-phosphate aminotransferase is an enzyme responsible for a reaction of producing glucosamine 6-phosphate from fructose 6-phosphate, and is encoded by the glmS gene. The glmS gene is preferably derived from Escherichia coli. Since glucosamine 6-phosphate is an intermediate in the NeuAc synthesis pathway, Glms enhances supply of NeuAc.

NanT is a NeuAc transporter that takes up excreted NeuAc into the bacterial cell, thereby enabling reuse of NeuAc. The nanT gene encoding the NeuAc transporter is preferably derived from Escherichia coli.

CMP-NeuAc synthase is an enzyme responsible for a reaction of producing CMP-NeuAc using cytidine-5'-triphosphate (hereinafter, also referred to as "CTP") and NeuAc as a substrate. Examples of the DNA encoding CMP-NeuAc synthase include the neuA gene preferably derived from a prokaryote such as bacteria or a yeast, particularly preferably derived from a prokaryote, and most preferably derived from the Pasteurella multocida strain PM70.

Lactose permease is a membrane protein that takes up lactose into cells and is encoded by the lacY gene. The lacY gene is preferably derived from Escherichia coli. When lactose is added from the outside, lactose permease promotes the uptake of lactose into the bacterial cells.

Glucosamine acetyltransferase is an enzyme responsible for a reaction of producing GlcNAc from glucosamine 6-phosphate. Glucosamine acetyltransferase derived from Saccharomyces cerevisiae is encoded by the GNA1 gene.

N-acetylmannosamine epimerase is associated with epimerization of GlcNAc to ManNAc. N-acetylmannosamine epimerase derived from the cyanobacterium Synechocystis sp. PCC6803 is encoded by the slr1975 gene.

Therefore, in one embodiment of the present invention, the parent strain is preferable a genetically modified microorganism preferably comprising at least one nucleotide sequence selected from the neuC gene (SEQ ID NO: 19), the neuB gene (SEQ ID NO: 18 or ADE86687.1), the pyc gene (WP_010970538.1 or WP_208400776.1), the glmS gene (Accession No.: BAE77559.1), the nanT gene (SEQ ID NO: 15 or BAE77267.1), the neuA gene (SEQ ID NO: 20), the lacY gene (Accession No.: BAE76125.1), the GNA1 gene (NP_116637.1), and the slr1975 gene (BAK50383.1). In one embodiment of the present invention, the genetically modified microorganism preferably has an enhanced 3'SL productivity as compared with that of a parent strain that is not genetically modified.

As a method for producing Escherichia coli having at least one activity selected from an UDP-GlcNAc epimerase activity, a NeuAc synthase activity, a pyruvate carboxylase activity, an L-glutamine-D-fructose 6-phosphate aminotransferase activity, a NanT activity, a CMP-NeuAc synthase activity, a lactose permease activity, a glucosamine acetyltransferase activity, and an N-acetylmannosamine epimerase activity, or an enhanced activity thereof, a known method may be used. Specific examples thereof include methods using various genetic manipulations (Syst Microbiol Biomanufact, 2021, 1, 291).

### <<Preferred Activity to be Reduced or Deleted in Parent Strain>>

In the parent strain, at least one activity selected from an N-acetylglucosamine (hereinafter, also referred to as "GlcNAc") transporter activity, an N-acetylmannosamine kinase activity, an N-acetylglucosamine-6-phosphate-2-epimerase activity, an RNase adapter protein activity, a transcriptional regulator activity that negatively regulates transcription of a gene group associated with NeuAc assimilation, a NeuAc lyase activity, a β-galactosidase activity, and a galactoside acetyltransferase activity is preferably reduced or deleted, and among them, a β-galactosidase activity and a NeuAc lyase activity are particularly preferably reduced or deleted.

The GlcNAc transporter is involved in the uptake and assimilation of GlcNAc into the bacterial cell, and is encoded by the nagE gene and the nagB gene. The reduction or deletion of the GlcNAc transporter activity can promote production of GlcNAc, which is a substrate for ManNAc.

N-acetylmannosamine kinase is an enzyme responsible for a reaction of producing N-acetylmannosamine 6-phosphate (hereinafter, also referred to as "ManNAc-6P") from ManNAc, and is encoded by the nanK gene. By preventing production of a substance other than NeuAc from ManNAc, the supply of NeuAc can be promoted.

N-acetylglucosamine-6-phosphate-2-epimerase is an enzyme responsible for a reaction of converting ManNAc-6P to N-acetyl-D-glucosamine 6-phosphate (hereinafter, also referred to as "GlcNAc-6P"), and is encoded by the nanE gene.

An RNase adapter protein is a transcription factor that negatively regulates an activity of a gene encoding an enzyme associated with NeuAc biosynthesis and is encoded by the yhbJ gene. The yhbJ gene is also called the rapZ gene. YhbJ is a transcriptional regulator that negatively regulates an activity of the glmS gene encoding L-glutamine-D-fructose 6-phosphate aminotransferase associated with NeuAc biosynthesis.

The transcriptional regulator that negatively regulates transcription of a gene group associated with the NeuAc assimilation is involved in the synthesis and metabolism of sialic acid, and is encoded by the nanR gene.

NeuAc lyase is an enzyme that decomposes NeuAc into ManNAc and pyruvic acid, and is encoded by the nanA gene.

β-galactosidase is an enzyme that hydrolyzes lactose and is encoded by the lacZ gene.

Galactoside acetyltransferase is an enzyme having an activity of acetylating lactose or analogues thereof, and is encoded by the lacA gene.

Therefore, in one embodiment of the present invention, the parent strain is more preferably a genetically modified microorganism not comprising at least one nucleotide sequence selected from the nagE gene, the nagB gene, the nanK gene, the nanE gene, the yhbJ gene, the nanR gene, the nanA gene, the lacZ gene, and the lacA gene. In one embodiment of the present invention, the genetically modified microorganism preferably has an enhanced 3'SL productivity as compared with that of a parent strain that is not genetically modified.

As a method for producing Escherichia coli in which at least one activity selected from a GlcNAc transporter activity, an N-acetylmannosamine kinase activity, an N-acetylglucosamine-6-phosphate-2-epimerase activity, an RNase adapter protein activity, a transcriptional regulator activity that negatively regulates transcription of a gene group associated with the NeuAc assimilation, a NeuAc lyase activity, a β-galactosidase activity, and a galactoside acetyltransferase activity is reduced or deleted, a known method may be used. Specific examples thereof include methods using various genetic manipulations (Metabolic Engineering, 2017, 41: 23-38).

### <Acquisition of Microorganism having Enhanced Activity of Specific Protein>

Examples of the microorganism having an enhanced activity of the protein according to any one of the above [A] and [1] to [3] as compared with the microorganism of the parent strain include a microorganism having an increased copy number of the gene as compared with the parent strain, which is obtained by transforming the microorganism of the parent strain with a recombinant DNA containing a DNA encoding the protein. In the present description, increasing the copy number of the gene may mean that the gene is newly held in a parent strain that does not carry the gene on a chromosome or a plasmid, or that the gene is additionally held in a strain that carries the gene on a chromosome or a plasmid.

Examples of the microorganism having an increased copy number of the gene as compared with the parent strain, which is obtained by transforming the microorganism of the parent strain with a recombinant DNA containing a DNA encoding the protein according to any one of the above [A] and [1] to [3] include a microorganism in which the copy number of the gene on the chromosomal DNA is increased by transforming the microorganism of the parent strain with a recombinant DNA containing a DNA encoding the protein according to any one of the above [A] and [1] to [3], and a microorganism in which the gene is carried outside of the chromosomal DNA as a plasmid DNA.

The DNA encoding the protein according to any one of the above [A] and [1] to [3] may be any DNA as long as it encodes a protein having an activity of the protein according to any one of the above [A] and [1] to [3]. Specific examples thereof include one DNA selected from the group consisting of the following [4] to [7].
[4] A DNA encoding the protein according to any one of the above [A] and [1] to [3]
[5] A DNA consisting of the nucleotide sequence represented by SEQ ID NO: 3, 5, 7, or 9
[6] A DNA hybridizing with a DNA consisting of a nucleotide sequence complementary to the nucleotide sequence represented by SEQ ID NO: 3, 5, 7, or 9 under stringent conditions and encoding a homologous protein having an α2,3-sialyltransferase activity
[7] A DNA consisting of a nucleotide sequence having an identity of 95% or more, preferably 97% or more, more preferably 98% or more, and most preferably 99% or more with the nucleotide sequence represented by SEQ ID NO: 3, 5, 7, or 9 and encoding a homologous protein having an α2,3-sialyltransferase activity

In the above [6], the term "hybridizing" means that a DNA hybridizes to a DNA having a specific nucleotide sequence or a part of the DNA. Accordingly, the DNA having the specific nucleotide sequence or a part thereof is a DNA that can be used as a probe for Northern or Southern blot analysis, or a DNA that can be used as an oligonucleotide primer for PCR analysis.

Examples of the DNA used as a probe include a DNA having at least 100 bases or more, preferably 200 bases or more, and more preferably 500 bases or more. Examples of the DNA used as a primer include a DNA having at least 10 bases or more, and preferably 15 bases or more.

A method for a DNA hybridization experiment is well known, and for example, those skilled in the art can determine hybridization conditions according to the present description. The hybridization conditions can be determined according to those described in Molecular Cloning, 4th Edition (2012), Methods for General and Molecular Bacteriology, ASM Press

(1994), and Immunology methods manual, Academic press (1996) as well as many other standard textbooks.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mmol/L sodium chloride, 75 mmol/L sodium citrate), 50 mmol/L sodium phosphate (pH 7.6), a 5× Denhardt's solution, 10% dextran sulfate, and a 20 µg/l of denatured salmon sperm DNA, and then the filter is washed in a 0.2×SSC solution at, for example, about 65°C.

Examples of the DNA capable of hybridizing under the above stringent conditions include DNAs having an identity of at least 95% or more, preferably 97% or more, more preferably 98% or more, and most preferably 99% or more with the DNA consisting of the nucleotide sequence represented by SEQ ID NO: 3, 5, 7, or 9 when calculated based on the parameters or the like using BLAST, FASTA, or the like.

The DNA encoding the protein of the above [1] and the DNA in the above [5] can be obtained by, for example, Southern hybridization for a chromosomal DNA library of a microorganism, preferably a microorganism belonging to the genus Escherichia, and more preferably an Escherichia coli BW25113 strain using a probe DNA that can be designed based on the nucleotide sequence represented by SEQ ID NO: 3, 5, 7, or 9, or PCR [PCR protocols, Academic Press (1990)] using, as a template, a chromosomal DNA of a microorganism and using a primer DNA that can be designed based on the nucleotide sequence.

The DNA encoding the mutant protein of the above [2] can be obtained by, for example, performing error-prone PCR, or the like, using, as a template, a DNA consisting of the nucleotide sequence represented by SEQ ID NO: 3, 5, 7, or 9.

The DNA encoding the mutant protein in the above [2] can be obtained by PCR using a set of PCR primers each having, at the 5' end thereof, a nucleotide sequence designed to insert a target mutation (deletion, substitution, insertion, or addition) [Gene, 77, 51 (1989)].

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced is obtained.

The DNA encoding the homologous protein of the above [3] and the DNA of the above [6] and [7] can be obtained, for example, by a method same as the above method for obtaining the DNA by, for example, searching various gene sequence databases for a nucleotide sequence having an identity of 95% or more, preferably 97% or more, further preferably 98% or more, and most preferably 99% or more with the nucleotide sequence represented by SEQ ID NO: 3, 5, 7, or 9 searching various protein sequence databases for an amino acid sequence having an identity of 90% or more, preferably 95% or more, more preferably 97% or more, further preferably 98% or more, and most preferably 99% or more with the amino acid sequence represented by SEQ ID NO: 4, 6, 8, or 10, and using a probe DNA or a primer DNA that can be designed based on the nucleotide sequence or the amino acid sequence obtained by the search, and a microorganism having the DNA.

The nucleotide sequence of the obtained DNA according to any one of the above [4] to [7] can be determined by using the DNA as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a nucleotide sequence analyzer such as a 3700 DNA analyzer (manufactured by Applied Biosystems).

The host cell that can be used for determining the nucleotide sequence of the DNA may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990).

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial-length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

The recombinant DNA containing the DNA encoding the protein according to any one of the above [A] and [1] to [3] refers to a recombinant DNA obtained by incorporating the DNA into an expression vector which is autonomously replicable in a parent strain or can be incorporated into a chromosome and contains a promoter at a position where the DNA can be transferred.

When the recombinant DNA is a recombinant DNA capable of being incorporated into a chromosome, the recombinant DNA may not contain a promoter.

The microorganism having an increased copy number of the gene as compared with the parent strain, which is obtained by transforming the microorganism of the parent strain with the recombinant DNA containing the DNA encoding the protein according to any one of the above [A] and [1] to [3] can be obtained by the following method.

Based on the DNA encoding the protein according to any one of the above [A] and [1] to [3] and obtained by the above method, a DNA fragment of an appropriate length containing a portion encoding the protein is prepared as necessary. A transformant having improved productivity can be obtained by substituting a base such that a nucleotide sequence of the portion encoding the protein becomes an optimal codon for expression in a host cell.

A recombinant DNA is prepared by inserting the DNA fragment downstream of a promoter of an appropriate expression vector. By transforming the parent strain with the recombinant DNA, a microorganism having an increased copy number of a gene encoding the protein as compared with the parent strain can be obtained.

When a prokaryote such as bacteria is used as a parent strain, the recombinant DNA is preferably a recombinant DNA composed of a promoter, a ribosomal binding sequence, the DNA according to any one of the above [4] to [7], and a transcription termination sequence. A gene for regulating the promoter may be contained.

It is preferable to use a plasmid in which a distance between a Shine-Dalgarno sequence, which is a ribosome binding sequence, and a start codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

An expression level of the protein having an α2,3-sialyltransferase activity can be improved by substituting a base such that the nucleotide sequence of the portion encoding the protein having an α2,3-sialyltransferase activity becomes an optimal codon for host expression. Examples of the protein having an α2,3-sialyltransferase activity include the protein according to any one of the above [A] and [1] to [3]. Information on the frequency of codon use in the parent strain used in the production method according to the present invention can be obtained through a public database.

The expression vector is not particularly limited as long as it is an appropriate nucleic acid molecule for introducing the target DNA into a host and causing the target DNA to be amplified and expressed. For example, not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW119 (manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK(+), pBluescript II KS(-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p6378-6385], pPAC31 (WO1998/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from, for example, an Escherichia coli or a phage, such as a uspA promoter, a lac promoter, a PL promoter, a PR promoter, or a PSE promoter can be used. Examples thereof include a promoter which is artificially modified in design, such as a promoter with two trp promoters in series, a tac promoter, a trc promoter, a lacT7 promoter, or a letI promoter.

When a microorganism belonging to the genus Corynebacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

When the above expression vector is used, the promoter may be any promoter as long as it functions in cells of microorganisms belonging to the genus Corynebacterium, and for example, a P54-6 promoter [Appl. Microbiol. Biotechnol., 53, 674-679 (2000)] can be used.

When a yeast strain is used as the parent strain, examples of the expression vector include YEp13 (ATCC 37115), YEp24 (ATCC 37051), YCp50 (ATCC 37419), pHS19, and pHS15.

The promoter in the case of using the above expression vector may be any promoter as long as it functions in cells of the yeast strain, and examples thereof include a PHO5 promoter, a PGK promoter, a GAP promoter, an ADH promoter, a gal1 promoter, a gal10 promoter, a heat shock polypeptide promoter, an MFα1 promoter, and a CUP1 promoter.

By inserting the DNA fragment according to any one of the above [4] to [7] downstream of a promoter of an appropriate expression vector, a recombinant DNA to be used in the production method of the present invention can be prepared.

Examples of a method for introducing a recombinant DNA into a parent strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a host cell include a homologous recombination method. Examples of the homologous recombination method include a method using a plasmid for homologous recombination that can be prepared by ligating with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6640-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the recombinant DNA is introduced into the parent strain as an autonomously replicable plasmid or incorporated into a chromosome of the parent strain can be confirmed by, for example, a method in which a gene originally contained in a chromosomal DNA of a microorganism cannot be amplified, but the gene introduced through transformation can be amplified by PCR using a primer set to confirm an amplification product. The fact that the transcription amount of the DNA or the production amount of the protein encoded by the DNA is increased can be confirmed by a method of comparing the transcription amount of the gene in the microorganism with that of the parent strain by Northern blotting, or the production amount of the protein in the microorganism with that of the parent strain by Western blotting.

The fact that the microorganism produced by the above method is a microorganism having an enhanced activity of the protein according to any one of the above [A] and [1] to [3] as compared with the parent strain can be confirmed by appropriately diluting a culture solution after culturing the microorganism, centrifuging the culture solution, analyzing 3'SL contained in the supernatant by HPLC or the like, and comparing 3'SL with that of the parent strain.

The above-described microorganism has an enhanced activity of the protein according to any one of the above [A] and [1] to [3] as compared with that of the parent strain, and thus the transfer of sialic acid residues from CMP-sialic acid to the galactose at the terminal of the receptor carbohydrate via the α2,3 bond can be promoted, and the productivity of 3'SL can be improved. Examples of such a microorganism include tNlsiaTΔN20AA, tNlsiaTΔN23AA, tNlsiaTΔN27AA, and tNlsiaTΔN37AA, which have enhanced expression of the α2,3-sialyltransferase gene to be described later in Examples.

An example of such a microorganism is a microorganism having an enhanced α2,3-sialyltransferase activity, which can improve 3'SL productivity due to a high activity of an α2,3-sialyltransferase.

### 2. Production Method of 3'SL

An example of a method for producing 3'SL of one aspect of the present invention is as follows.

A method for producing 3'SL, including: preparing the microorganism described above in 1.; and producing 3'SL in a culture using the microorganism.

The method for culturing the microorganism described above in 1. can be performed according to a usual method used for culturing a microorganism.

As a culture medium for culturing a microorganism, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

As the microorganism of the present invention used in the method for producing 3'SL, a microorganism having an ability to produce a receptor carbohydrate such as glucose, lactose, or lactose monohydrate may be used.

In the method for producing 3'SL, glucose, liquid sugar, sucrose, lactose, NeuAc, and the like may be added to the medium during culture.

In the method for producing 3'SL, instead of adding lactose, NeuAc, and the like to the medium during culture, lactose, NeuAc, and the like may be supplied to the microorganism of one aspect of the present invention by simultaneously culturing a microorganism capable of producing lactose and NeuAc with the microorganism of the present invention.

In the method for producing an oligosaccharide, it is preferable that the medium is free of GlcNAc transporter, N-acetylmannosamine kinase, N-acetylglucosamine-6-phosphate-2-epimerase, RNase adapter protein, a transcriptional regulator that negatively regulates the transcription of a gene group associated with the NeuAc assimilation, NeuAc lyase, β-galactosidase, and galactoside acetyltransferase.

Generally, the culture is preferably performed under aerobic conditions by agitation culture with aeration. The culture temperature is generally 28°C to 37°C, and the culture time is generally 36 hours to 4 days. The pH of the culture solution during culture is generally maintained at 6.4 to 7.4. The pH is adjusted using an inorganic or organic acid, an alkaline solution, urea, calcium carbonate, ammonia, or the like.

3'SL can be produced by producing and accumulating 3'SL in the culture by the above-described culture and collecting the 3'SL from the culture.

Generally, 3'SL can be collected from a supernatant after centrifugation of the culture. When 3'SL is accumulated in bacterial cells, 3'SL can be collected by using an ion exchange resin method or the like from a supernatant obtained by, for example, crushing the bacterial cells with ultrasonic waves or the like and removing the bacterial cells by centrifugation.

### [Analysis Example]

In Examples, analysis and quantification of 3'SL were performed by the following procedure. After the culture, the culture solution containing the microorganism was appropriately diluted and sterilized by filter, and the supernatant was collected. 3'SL contained in the supernatant was analyzed by an analyzer SPD-20A (manufactured by Shimadzu Corporation).

### [Analysis Conditions]

Column: Shodex SUGAR SH1011
Column temperature: 50°C
Eluent composition: 5mM sulfuric acid aqueous solution
Flow rate: 0.6 ml/min
Detector: SPD-20A

### [Example]

### [Example 1] Construction of Microorganism Used for 3'SL Production

### (1) Construction of 3'SL Production Host

### <Escherichia Coli in which yhbJ Gene was Deleted>

Escherichia coli in which the transcriptional regulator yhbJ gene (SEQ ID NO: 11) for negatively regulating an activity of the glmS gene encoding L-glutamine-D-fructose 6-phosphate aminotransferase associated with biosynthesis of N-acetylneuraminic acid (hereinafter, referred to as NeuAc) was disrupted was prepared by the following procedure.

A BW25113 ΔyhbJ strain in which the yhbJ gene was completely deleted was obtained by the same method as in Baba et al. [Baba T. et al. (2006) Mol systems Biol] using the BW25113 strain (Keio collection (Systematic single-gene knock-out mutants of E. coli K-12)) as a host.

### <Acquisition of DNA Fragment to be Used as Marker for Gene Deletion>

PCR was performed using, as a primer set, a DNA consisting of the nucleotide sequences represented by "Primer set" in Table 1 and using, as a template, a DNA described in "Template" in Table 1 to obtain each amplified DNA fragment.

### [Table 1]

**Table 1**

| Primer set (SEQ ID NO:) | Template | Amplified DNA fragment |
|---|---|---|
| 21 and 22 | pHSG396 (manufactured by Takara Bio Inc.) | cat |
| 23 and 24 | Genomic DNA of Bacillus subtilis 168 strain | sacB |

A genomic DNA of the Bacillus subtilis 168 strain was prepared by an ordinary method. The cat of the amplified DNA fragment comprises about 200 bp upstream to about 50 bp downstream of the cat gene (encoding chloramphenicol acetyltransferase) on pHSG396. The sacB of the amplified DNA fragment comprises about 300 bp upstream to about 100 bp downstream of the sacB gene (encoding levansucrase) on the genomic DNA of the Bacillus subtilis 168 strain.

Next, PCR was performed using, as a template, a mixture of the cat and sacB of the amplified DNA fragment in an equimolar ratio and using, as a primer set, a DNA consisting of the nucleotide sequences represented by SEQ ID NOs: 21 and 24 to obtain a DNA fragment (hereinafter, referred to as cat-sacB) containing the cat gene and the sacB gene.

### <Escherichia Coli in which lacZ Gene was Deleted>

Escherichia coli in which the lacZ gene (SEQ ID NO: 12) encoding β-galactosidase associated with decomposition of lactose was disrupted was prepared by the following procedure using the BW25113ΔyhbJ strain constructed above as a parent strain.

PCR was performed using, as a template, the chromosomal DNA of the BW25113 strain prepared by an ordinary method and using, as a primer set, a DNA consisting of the nucleotide sequences represented by "Primer set" in Table 2 to obtain each amplified DNA fragment.

### [Table 2]

**Table 2**

| Primer set (SEQ ID NO:) | Amplified DNA fragment | Remarks |
|---|---|---|
| 25 and 26 | lacZ upstream 1 | Nucleotide sequence represented by SEQ ID NO: 21 is complementary to nucleotide sequence at 5' end of nucleotide sequence represented by SEQ ID NO: 26. |
| 27 and 28 | lacZ downstream 1 | Nucleotide sequence represented by SEQ ID NO: 24 is complementary to nucleotide sequence at 5' end of nucleotide sequence represented by SEQ ID NO: 27. |
| 29 and 30 | lacZ upstream 2 | Nucleotide sequence represented by SEQ ID NO: 30 is complementary to nucleotide sequence represented by SEQ ID NO: 31. |
| 31 and 32 | lacZ downstream 2 | |

The lacZ upstream 1 and lacZ upstream 2 each comprise about 700 bp upstream from a start codon of the lacZ gene. The lacZ downstream 1 and lacZ downstream 2 each comprise about 800 bp downstream from a stop codon of the lacZ gene.

PCR was performed using, as a template, a mixture of the lacZ upstream 1, the lacZ downstream 1, and the cat-sacB fragment in an equimolar ratio and using, as a primer set, a DNA consisting of the nucleotide sequences represented by SEQ ID NOs: 33 and 34 to obtain a DNA fragment (hereinafter, referred to as lacZ::cat-sacB) consisting of a sequence with the cat-sacB fragment inserted in a region around the lacZ gene.

PCR was performed using, as a template, a mixture of the lacZ upstream 2 and the lacY downstream 2 in an equimolar ratio and using, as a primer set, a DNA consisting of the nucleotide sequences represented by SEQ ID NOs: 33 and 34 to obtain a DNA fragment (hereinafter, referred to as ΔlacZ) consisting of a sequence with the lacZ upstream and the lacZ downstream directly ligated to each other without lacZ.

The lacZ::cat-sacB fragment was introduced into a BW25113ΔyhbJ strain carrying a plasmid pKD46 containing a gene encoding λ recombinase [Datsenko, K. A., Warner, B. L. L., Proc. Natl. Acad.Sci, USA, Vol. 97, 6640-6645, (2000)] by an electroporation method to obtain a transformant (a transformant with the lacZ gene substituted with lacZ::cat-sacB) exhibiting chloramphenicol resistance and sucrose sensitivity.

The ΔlacZ fragment was introduced into the transformant by the electroporation method to obtain a transformant (a transformant with lacZ::cat-sacB substituted with ΔlacZ) exhibiting chloramphenicol sensitivity and sucrose resistance. The transformant was named BW25113ΔyhbJΔlacZ strain.

### <Escherichia Coli in which nanR, nanA, nanT, nanE, and nanK Genes were Deleted>

Escherichia coli in which the nanR gene (SEQ ID NO: 13) encoding a transcriptional regulator that negatively regulates the transcription of a gene group associated with NeuAc assimilation, the nanA gene (SEQ ID NO: 14) encoding NeuAc lyase, the nanT gene (SEQ ID NO: 15) encoding NeuAc transporter, the nanE gene (SEQ ID NO: 16) encoding N-acetylglucosamine-6-phosphate-2-epimerase, and the nanK gene (SEQ ID NO: 17) encoding N-acetylmannosamine kinase were disrupted was prepared by the following procedure using the BW25113ΔyhbJΔlacZ strain as a host. nanR, NanA, NanT, NanE, and NanK (hereinafter, referred to as NanRATEK) form an operon on the Escherichia coli genome.

PCR was performed using, as a template, the chromosomal DNA of the BW25113 strain prepared by an ordinary method and using, as a primer set, a DNA consisting of the nucleotide sequences represented by "Primer set" in Table 3 to obtain each amplified DNA fragment.

### [Table 3]

**Table 3**

| Primer set (SEQ ID NO:) | Amplified DNA fragment | Remarks |
|---|---|---|
| 35 and 36 | nanR upstream 1 | Nucleotide sequence represented by SEQ ID NO: 21 is complementary to nucleotide sequence at 5' end of nucleotide sequence represented by SEQ ID NO: 36. |
| 37 and 38 | nanK downstream 1 | Nucleotide sequence represented by SEQ ID NO: 24 is complementary to nucleotide sequence at 5' end of nucleotide sequence represented by SEQ ID NO: 37. |
| 39 and 40 | nanR upstream 2 | Nucleotide sequence represented by SEQ ID NO: 40 is complementary to nucleotide sequence represented by SEQ ID NO: 41. |
| 41 and 42 | nanK downstream 2 | |

The nanR upstream 1 and nanR upstream 2 each comprise about 800 bp upstream from a start codon of the nanR gene. The nanK downstream 1 and nanK downstream 2 each comprise 850 bp downstream from a stop codon of the nanK gene.

PCR was performed using, as a template, a mixture of the nanR upstream 1, the nanK downstream 1, and the cat-sacB fragment in an equimolar ratio and using, as a primer set, a DNA consisting of the nucleotide sequences represented by SEQ ID NOs: 43 and 44 to obtain a DNA fragment (hereinafter, referred to as nanRATEK::cat-sacB) consisting of a sequence with the cat-sacB fragment inserted into a sequence in a region around the nanRATEK gene.

PCR was performed using, as a template, a mixture of the nanR upstream 2 and the nanK downstream 2 in an equimolar ratio and using, as a primer set, a DNA consisting of the nucleotide sequences represented by SEQ ID NOs: 43 and 44 to obtain a DNA fragment (hereinafter, referred to as ΔnanRATEK) consisting of a sequence with the nanR upstream and the nanK downstream directly ligated to each other without the nanRATEK.

The nanRATEK::cat-sacB fragment was introduced into the BW25113ΔyhbJΔlacZ strain carrying a plasmid pKD46 containing a gene encoding λ recombinase by an electroporation method to obtain a transformant (a transformant with the nanRATEK gene substituted with nanRATEK::cat-sacB) exhibiting chloramphenicol resistance and sucrose sensitivity.

The ΔnanRATEK fragment was introduced into the transformant by an electroporation method to obtain a transformant (a transformant with nanRATEK::cat-sacB substituted with ΔnanRATEK) exhibiting chloramphenicol sensitivity and sucrose resistance. The transformant was named BW25113ΔyhbJΔlacZΔnanRATEK strain.

### (2) Construction of NeuAc Production Plasmid

A plasmid expressing the NeuAc synthase gene CjneuB (SEQ ID NO: 18) derived from Campylobacter jejuni ATCC 43438 strain and FpneuC (SEQ ID NO: 19) encoding UDP-GlcNAc epimerase derived from Flavobacterium psychrophilum NBRC100250 strain was prepared by the following procedure.

PCR was performed using, as a primer set, a DNA consisting of the nucleotide sequences represented by "Primer set" in Table 4 and using, as a template, a DNA described in "Template" in Table 4 to obtain amplified DNA fragments.

Since a start codon in the wild-type sequence of the FpneuC (SEQ ID NO: 19) is GTG, ATG was added to the primer sequence such that the start codon of the amplified DNA fragment FpneuC obtained by PCR was ATG in order to express the primer sequence in Escherichia coli.

### [Table 4]

**Table 4**

| Primer set (SEQ ID NO:) | Template | Amplified DNA fragment |
|---|---|---|
| 45 and 46 | Genomic DNA of Campylobacter jejuni ATCC 43438 strain | CjneuB (SEQ ID NO: 18) |
| 47 and 48 | Genomic DNA of Flavobacterium psychrophilum NBRC100250 strain | FpneuC (SEQ ID NO: 19) |

Genomic DNAs of the Campylobacter jejuni ATCC 43438 strain and the Flavobacterium psychrophilum NBRC100250 strain were prepared by an ordinary method.

The nucleotide sequences represented by SEQ ID NOs: 46 and 47 each comprise a complementary sequence at the 5' end.

PCR was performed using, as a template, a mixture of the CjneuB fragment and FpneuC fragment obtained as described above in an equimolar ratio and using, as a primer set, a DNA consisting of the nucleotide sequences represented by SEQ ID NOs: 45 and 48 to obtain a DNA fragment (hereinafter, referred to as a CjneuB-FpneuC fragment) of about 2.2 kb in which the CjneuB fragment and the FpneuC fragment were ligated.

The CjneuB-FpneuC fragment and the expression vector pTrc99a (manufactured by GE Healthcare Bioscience) were ligated to each other using an In-Fusion HD Cloning Kit (manufactured by Takara Bio Inc.) to obtain an expression plasmid pTrc99a-CjneuB-FpneuC (hereinafter, referred to as pBC).

### (3) Construction of 3'SL Production Plasmid

A plasmid expressing the full-length or truncated sialyltransferase gene NlsiaT (SEQ ID NO: 1, 3, 5, 7, or 9) derived from Neisseria lactamica ATCC 23970 strain and the CMP-NeuAc synthase gene PmneuA (SEQ ID NO: 20) derived from Pasteurella multocida subsp. multocida str. Pm70 ATCC BAA-1909 strain was prepared by the following procedure.

PCR was performed using the chromosomal DNA of Neisseria lactamica prepared by an ordinary method as a template and a DNA consisting of the nucleotide sequence represented by "Primer set" in Table 5 as a primer set to obtain each amplified DNA fragment.

### [Table 5]

**Table 5**

| Primer set (SEQ ID NO:) | Amplified DNA fragment | Remarks |
|---|---|---|
| 49 and 50 | NlsiaT (SEQ ID NO: 1) | Full-length NlsiaT. |
| 51 and 50 | tNlsiaTΔ(delta)N20AA (SEQ ID NO: 3) | Truncated NlsiaT in which 20 amino acids are removed from N-terminus of NlsiaT represented by SEQ ID NO: 2. |
| 52 and 50 | tNlsiaTΔ(delta)N23AA (SEQ ID NO: 5) | Truncated NlsiaT in which 23 amino acids are removed from N-terminus of NlsiaT represented by SEQ ID NO: 2. |
| 53 and 50 | tNlsiaTΔ(delta)N27AA (SEQ ID NO: 7) | Truncated NlsiaT in which 27 amino acids are removed from N-terminus of NlsiaT represented by SEQ ID NO: 2. |
| 54 and 50 | tNlsiaTΔ(delta)N37AA (SEQ ID NO: 9) | Truncated NlsiaT in which 37 amino acids are removed from N-terminus of NlsiaT represented by SEQ ID NO: 2. |

PCR was performed using the chromosomal DNA of Pasteurella multocida subsp. multocida str. Pm70 ATCC BAA-11909 prepared by an ordinary method as a template and a DNA consisting of the nucleotide sequences represented by SEQ ID NOs: 55 and 56 as a primer set to obtain a PmneuA fragment.

The nucleotide sequences represented by SEQ ID NOs: 50 and 55 each comprise a complementary sequence at the 5' end.

PCR was performed using a mixture of the NlsiaT fragment, tNlsiaTΔN20AA fragment, tNlsiaTAN23AA fragment, tNlsiaTΔN27AA fragment or tNlsiaTΔN37AA fragment, and PmneuA fragment obtained as described above in an equimolar ratio as a template and a DNA consisting of the nucleotide sequences represented by SEQ ID NOs: 49, 51, 52, 53 or 54, and 56 as a primer set to obtain DNA fragments in which the NlsiaT fragment and the PmneuA fragment were ligated.

The above-obtained DNA fragments in which the NlsiaT fragment and the PmneuA fragment were ligated and the expression vector pSTV29 (manufactured by Takara Bio Inc.) were ligated using an In-Fusion HD Cloning Kit (manufactured by Takara Bio Inc.) to obtain expression plasmids pSTV-NlsiaT, pSTV-tNlsiaTΔN20AA, pSTV-tNlsiaTΔN23AA, pSTV-tNlsiaTΔN27AA and pSTV-tNlsiaTΔN37AA.

### (4) Construction of Microorganism having NeuAc Production Plasmid

The BW25113ΔyhbJΔlacZΔnanRATEK strain constructed in the above (1) was transformed with the NeuAc production plasmid pBC obtained in the above (2) to construct Escherichia coli carrying pBC, which was named BW25113ΔyhbJΔlacZΔnanRATEK/pBC strain.

### (5) Construction of Microorganism having 3'SL Production Plasmid

The BW25113ΔyhbJΔlacZΔnanRATEK/pBC strain constructed in the above (4) was transformed with the 3'SL production plasmids obtained in the above (2), pSTV-NlsiaT, pSTV-tNlsiaTΔN20AA, pSTV-tNlsiaTΔN23AA, pSTV-tNlsiaTΔN27AA and pSTV-tNlsiaTAN37AA to construct Escherichia coli strains having various plasmids, which were named NlsiaT strain, tNlsiaTΔN20AA strain, tNlsiaTΔN23AA strain, tNlsiaTΔN27AA strain and tNlsiaTΔN37AA strain, respectively.

### [Example 2] Production of 3'SL according to Fermentation Method

The productivity of 3'SL of the NlsiaT strain, tNlsiaTΔN20AA strain, tNlsiaTΔN23AA strain, tNlsiaTΔN27AA strain, and tNlsiaTΔN37AA strain obtained in Example 1 was evaluated.

Each strain was cultured overnight at 30°C on an LB plate containing 100 mg/L ampicillin and 25 mg/L chloramphenicol, then inoculated into a large-sized test tube containing 5 ml of LB medium containing 100 mg/L ampicillin and 25 mg/L chloramphenicol, and cultured with shaking at 30°C for 16 hours.

Thereafter, 0.3 ml of the culture solution was inoculated into a large-sized test tube containing 3 ml of a production medium containing 100 mg/L ampicillin and 25 mg/L chloramphenicol [glycerol 30g/L, lactose 10 g/L, dipotassium hydrogen phosphate 16 g/L, potassium dihydrogen phosphate 14 g/L, ammonium sulfate 2.0 g/L, citric acid monohydrate 1.0 g/L, casamino acid (manufactured by Difco Corporation) 5.0 g/L, vitamin B1 10 mg/L, iron sulfate heptahydrate 50 mg/L, and manganese sulfate heptahydrate 10 mg/L (adjusted to pH 7.2 by aqueous sodium hydroxide, and then autoclaved)], [glycerol, lactose, and magnesium sulfate heptahydrate aqueous solution were prepared separately, autoclaved, cooled and then mixed], followed by culturing with shaking at 30°C for 24 hours. Five hours after the start of culture, isopropyl-β-thiogalactopyranoside (IPTG) was added such that a final concentration was 1 mM.

After completion of the culture, the culture solution was appropriately diluted, and the sample after filter sterilization was subjected to HPLC analysis to quantify a concentration of 3'SL contained in the supernatant. The results are shown in Table 6.

### [Table 6]

**Table 6**

| No. | Strain | 3'SL [g/L] |
|---|---|---|
| 1 | NlsiaT | 1.51±0.02 |
| 2 | tNlsiaTΔN20AA | 1.61±0.03 |
| 3 | tNlsiaTΔN23AA | 1.65±0.04 |
| 4 | tNIsiaTΔN27AA | 1.68±0.01 |
| 5 | tNIsiaTΔN37AA | 1.66±0.02 |

From the above results, it was revealed that when the truncated NlsiaT in which the N-terminus amino acids of the sialyltransferase NlsiaT derived from Neisseria lactamica ATCC 23970 strain were deleted was used, higher 3'SL productivity was observed than when the full-length NlsiaT was used. The effective lengths of N-terminus deletions were roughly equivalent at 20 amino acid residues, 23 amino acid residues, 27 amino acid residues, and 37 amino acid residues.

These truncated glycosyltransferases were shown to be more useful for efficient production of 3'SL than the full-length glycosyltransferases.

Although the present invention has been described in detail with reference to specific embodiments, it will be apparent to those skilled in the art that various changes and modifications can be made without departing from the spirit and scope of the present invention. The present application is based on a Japanese Patent Application No. 2022-091801 filed on June 6, 2022, the entire contents of which are incorporated herein by reference. All references cited herein are incorporated in their entirety.

### INDUSTRIAL APPLICABILITY

According to the present invention, 3'SL can be efficiently produced.

### SEQUENCE LISTING FREE TEXT

SEQ ID NO: 1: nucleotide sequence of sialyltransferase gene NlsiaT derived from Neisseria lactamica ATCC 23970 strain
SEQ ID NO: 2: amino acid sequence of sialyltransferase gene NlsiaT derived from Neisseria lactamica ATCC 23970 strain
SEQ ID NO: 3: nucleotide sequence of tNlsiaTΔN20AA
SEQ ID NO: 4: amino acid sequence of tNlsiaTΔN20AA
SEQ ID NO: 5: nucleotide sequence of tNlsiaTΔN23AA
SEQ ID NO: 6: amino acid sequence of tNlsiaTΔN23AA
SEQ ID NO: 7: nucleotide sequence of tNlsiaTΔN27AA
SEQ ID NO: 8: amino acid sequence of tNlsiaTΔN27AA
SEQ ID NO: 9: nucleotide sequence of tNlsiaTΔN37AA
SEQ ID NO: 10: amino acid sequence of tNlsiaTΔN37AA
SEQ ID NO: 11: yhbJ nucleotide sequence derived from Escherichia coli BW25113
SEQ ID NO: 12: lacZ nucleotide sequence derived from Escherichia coli BW25113
SEQ ID NO: 13: nanR nucleotide sequence derived from Escherichia coli BW25113
SEQ ID NO: 14: nanA nucleotide sequence derived from Escherichia coli BW25113
SEQ ID NO: 15: nanT nucleotide sequence derived from Escherichia coli BW25113
SEQ ID NO: 16: nanE nucleotide sequence derived from Escherichia coli BW25113
SEQ ID NO: 17: nanK nucleotide sequence derived from Escherichia coli BW25113
SEQ ID NO: 18: nucleotide sequence of NeuAc synthase gene CjneuB derived from Campylobacter jejuni ATCC 43438 strain
SEQ ID NO: 19: nucleotide sequence of FpneuC encoding UDP-GlcNAc epimerase derived from Flavobacterium psychrophilum NBRC100250 strain
SEQ ID NO: 20: nucleotide sequence of CMP-NeuAc synthase gene PmneuA derived from Pasteurella multocida subsp. multocida str. Pm70 ATCC BAA-1909 strain
SEQ ID NO: 21: nucleotide sequence of primer Fw for amplifying cat fragment
SEQ ID NO: 22: nucleotide sequence of primer Rv for amplifying cat fragment
SEQ ID NO: 23: nucleotide sequence of primer Fw for amplifying sacB fragment
SEQ ID NO: 24: nucleotide sequence of primer Rv for amplifying sacB fragment
SEQ ID NO: 25: nucleotide sequence of primer Fw for amplifying lacZ upstream 1 fragment
SEQ ID NO: 26: nucleotide sequence of primer Rv for amplifying lacZ upstream 1 fragment
SEQ ID NO: 27: nucleotide sequence of primer Fw for amplifying lacZ downstream 1 fragment
SEQ ID NO: 28: nucleotide sequence of primer Rv for amplifying lacZ downstream 1 fragment
SEQ ID NO: 29: nucleotide sequence of primer Fw for amplifying lacZ upstream 2 fragment
SEQ ID NO: 30: nucleotide sequence of primer Rv for amplifying lacZ upstream 2 fragment
SEQ ID NO: 31: nucleotide sequence of primer Fw for amplifying lacZ downstream 2 fragment
SEQ ID NO: 32: nucleotide sequence of primer Rv for amplifying lacZ downstream 2 fragment
SEQ ID NO: 33: nucleotide sequence of primer Fw for preparing lacZ disruption fragment
SEQ ID NO: 34: nucleotide sequence of a primer Rv for preparing lacZ disruption fragment
SEQ ID NO: 35: nucleotide sequence of primer Fw for amplifying nanR upstream 1 fragment
SEQ ID NO: 36: nucleotide sequence of primer Rv for amplifying nanR upstream 1 fragment
SEQ ID NO: 37: nucleotide sequence of primer Fw for amplifying nanK downstream 1 fragment
SEQ ID NO: 38: nucleotide sequence of primer Rv for amplifying nanK downstream 1 fragment
SEQ ID NO: 39: nucleotide sequence of primer Fw for amplifying nanR upstream 2 fragment
SEQ ID NO: 40: nucleotide sequence of primer Rv for amplifying nanR upstream 2 fragment
SEQ ID NO: 41: nucleotide sequence of primer Fw for amplifying nanK downstream 2 fragment
SEQ ID NO: 42: nucleotide sequence of primer Rv for amplifying nanK downstream 2 fragment
SEQ ID NO: 43: nucleotide sequence of primer Fw for preparing nanRATEK disruption fragment
SEQ ID NO: 44: nucleotide sequence of primer Rv for preparing nanRATEK disruption fragment
SEQ ID NO: 45: nucleotide sequence of primer Fw for amplifying CjneuB fragment
SEQ ID NO: 46: nucleotide sequence of primer Rv for amplifying CjneuB fragment
SEQ ID NO: 47: nucleotide sequence of primer Fw for amplifying FpneuC fragment
SEQ ID NO: 48: nucleotide sequence of primer Rv for amplifying FpneuC fragment
SEQ ID NO: 49: nucleotide sequence of primer Fw for amplifying NlsiaT
SEQ ID NO: 50: nucleotide sequence of primer Rv for amplifying NlsiaT
SEQ ID NO: 51: nucleotide sequence of primer Fw for amplifying tNlsiaTΔN20AA
SEQ ID NO: 52: nucleotide sequence of primer Fw for amplifying tNlsiaTΔN23AA
SEQ ID NO: 53: nucleotide sequence of primer Fw for amplifying tNlsiaTΔN27AA
SEQ ID NO: 54: nucleotide sequence of primer Fw for amplifying tNlsiaTΔN37AA
SEQ ID NO: 55: nucleotide sequence of primer Fw for amplifying PmneuA
SEQ ID NO: 56: nucleotide sequence of primer Rv for amplifying PmneuA

## Claims

1. A microorganism having an enhanced activity of a protein consisting of an amino acid sequence in which N-terminus 20 to 37 amino acid residues in the amino acid sequence represented by SEQ ID NO: 2 are deleted, wherein the microorganism has improved productivity of 3'-sialyllactose as compared with that of a parent strain.

2. The microorganism according to claim 1, wherein the protein consisting of an amino acid sequence in which N-terminus 20 to 37 amino acid residues in the amino acid sequence represented by SEQ ID NO: 2 are deleted is a protein according to any one of the following [1] to [3]:
[1] a protein consisting of the amino acid sequence represented by SEQ ID NO: 4, 6, 8, or 10,
[2] a mutant protein having an α2,3-sialyltransferase activity and consisting of an amino acid sequence in which 1 to 20 amino acids are deleted, substituted, inserted, or added in the amino acid sequence represented by SEQ ID NO: 4, 6, 8, or 10, and
[3] a homologous protein having an α2,3-sialyltransferase activity and consisting of an amino acid sequence having an identity of 90% or more with the amino acid sequence represented by SEQ ID NO: 4, 6, 8, or 10.

3. A method for producing 3'-sialyllactose, comprising: preparing the
microorganism according to claim 1 or 2; and producing 3'-sialyllactose in a culture using the microorganism.
